# EUROPÄISCHE PATENTSCHRIFT

(11) **EP 2 183 374 B1**
(45) Veröffentlichungstag und Bekanntmachung des Hinweises auf die Patenterteilung: **14.03.2012**
(21) Anmeldenummer: 08786369.2
(22) Anmeldetag: 23.07.2008
(51) Int. Cl.: C12P 5/02, C02F 11/04

(54) **Verfahren zur Konversion von Biomasse aus nachwachsenden Rohstoffen zu Biogas in anaeroben Fermentern**
Method for the conversion of biomass from renewable raw materials in anaerobic fermenters
Procédé pour convertir de la biomasse constituée de matières premières renouvelables en biogaz dans des fermenteurs anaérobies

(30) Priorität: 30.07.2007 DE 102007037202
(43) Veröffentlichungstag der Anmeldung: 12.05.2010
(73) Patentinhaber: Fraunhofer-Gesellschaft zur Förderung der angewandten Forschung e.V., 80686 München (DE)
(72) Erfinder: SCHWARZ, Björn, 01187 Dresden (DE); FASSAUER, Burkhardt, 01277 Dresden (DE); FRIEDRICH, Eberhard, 01445 Radebeul (DE); FRIEDRICH, Hannelore, 01445 Radebeul (DE); MICHAELIS, Alexander, 01217 Dresden (DE)
(74) Vertreter: Rauschenbach, Marion
(86) Internationale Anmeldenummer: PCT/EP2008/059677
(87) Internationale Veröffentlichungsnummer: WO 2009/016082

(56) Entgegenhaltungen:
- EP-A- 0 377 832
- EP-A- 1 978 086
- WO-A-2004/076082
- WO-A-2005/017091
- WO-A1-2006/042551

## Beschreibung

Die Erfindung bezieht sich auf die Gebiete der Biochemie und Energieerzeugung und betrifft ein Verfahren zur Konversion von Biomasse aus nachwachsenden Rohstoffen zu Biogas in anaeroben Fermentern, das in einer Biogaserzeugungsanlage eingesetzt wird, und dessen Einsatz sowohl bei der Monovergärung von nachwachsenden Rohstoffen als auch bei der Co-Vergärung mit Wirtschaftsdüngern (z.B. Gülle) in landwirtschaftlichen Biogasanlagen oder bei der Co-Vergärung mit Klärschlämmen auf kommunalen Kläranlagen möglich ist.

Die Umwandlung von Biomasse in energetisch zu verwertendes Biogas unter Ausnutzung der biochemischen Leistungsfähigkeit einer anaeroben Mischpopulation von Mikroorganismen wird großtechnisch sowohl in landwirtschaftlichen Biogasanlagen als auch in Faultürmen kommunaler Kläranlagen praktiziert. Die dabei verwendete Verfahrenstechnik umfasst ein sehr breites Spektrum an Kombinationen von Anzahl und Schaltung der Fermenter, Prozesstemperatur (mesophil, thermophil), Substratbehandlung, Beschickungsregime, Durchmischung, Aufenthaltsdauer und Raumbelastung.

Bei der Nutzung nachwachsender Rohstoffe als Haupt- oder Co-Substrat zur Biogaserzeugung verhindert deren chemische Struktur eine vollständige Umsetzung in Biogas. Größere Anteile des pflanzlichen Materials bestehen aus, für Mikroorganismen schwer oder gar nicht zugänglicher Cellulose, Hemicellulose und Lignin. Dies führt bei der Anwendung herkömmlicher Vergärungstechnologien, welche außer einer mechanischen Grobzerkleinerung des Inputmaterials keine weitergehende Substratbehandlung betreiben, zu unbefriedigenden und teilweise unwirtschaftlichen Abbaugraden. Die Verweilzeiten der Substrate in anaeroben Fermentern sind mit 50 bis 150 Tagen sehr lang und die Energieausbeute in Form von Biogas ist dennoch nicht befriedigend.

Des Weiteren ist bei in Reihe geschalteten Reaktoren (Kaskaden) nur der erste Reaktor voll ausgelastet, da der größte Anteil der mikrobiologisch verfügbaren organischen Stoffe bereits in den ersten 20 bis 30 Tagen umgesetzt wird. Alle nachgeschalteten Reaktoren sind in ihrer Abbauleistung und Geschwindigkeit sehr stark begrenzt. Ursache ist die sehr langsame Hydrolyse der verbliebenen organischen Fraktionen. Dieser die Geschwindigkeit limitierende Abbauschritt führt zu einer Unterbelastung der Methanogenese, welche noch deutliche Reserven aufweist.

Für die bessere Ausnutzung der primär in nachwachsenden Rohstoffen enthaltenen Energie bei der Vergärung ist die Hydrolyse, dass heißt, die Zerlegung komplexer Makromoleküle in ihre Grundbestandteile, extern zu beschleunigen. Dafür existiert nach dem Stand der Technik und vor allem auf dem Gebiet der Rohstoffgewinnung aus nachwachsenden Rohstoffen (Grundstoffchemie) eine Reihe von Verfahren. Verschiedene Kombinationen aus mechanischer Vorbehandlung (Zerkleinern, Mahlen), physikalischen (Dampfexplosionverfahren, Heißwasserbehandlung, Thermodruckhydrolyse), chemischen (Säuren, Laugen, Nassoxidation) und enzymatischen Methoden werden z.B. für eine Lyse von Cellulose und Hemicellulose eingesetzt. Die genannten Methoden lassen sich aber nicht direkt im Anwendungsgebiet der Biogaserzeuger einsetzten, da beispielsweise der Bedarf an Energie und Chemikalien für die zu behandelnden Volumenströme unwirtschaftlich hoch ist. Bei der Vergärung oder Co-Vergärung von nachwachsenden Rohstoffen werden dem Stand der Technik nach folgende Verfahren für eine Intensivierung des Abbaus eingesetzt:
- Mechanischer Voraufschluss (z.B. mittels Extruder)
   * hoher Feststoffgehalt des behandelten Substrates nötig →Einsatz vor ersten Fermenter für Feststoffsubstrate (Silagen),
   * geringe Beschleunigung des Abbaus im ersten Fermenter, welcher auch ohne Substrataufbereitung eine gute Abbauleistung besitzt,
- Enzymdosierung
   * spezialisiert auf wenige Substratgruppen (z.B. Cellulose),
   * geringe Steigerung des Abbaugrades
   *relativ hohe Kosten für Enzyme,
- Getrennte Hydrolyse/Methanisierung, Fest-Flüssigtrennung und pH-Steuerung durch Rückführung der Flüssigphase (EP 0 566 056 A1)
   * Entkopplung der festen, schwer hydrolysierbaren Stoffe vom Hauptstrom,
   * biologische Hydrolyse der abgetrennten Feststoffe in extra Fermenter →hydrolyse zu langsam und limitiert durch begrenztes Lysevermögen der Mikroorganismen,
- Thermische Desintegration zwischen in Reihe geschalteten ersten und zweiten Fermenter (DE 198 58 187 C2, DE 103 45 600 A1)
   * 10 bis 120 min bei 60 bis 90 °C → Temperaturen für Erhalt von Methanbakterien zu hoch und für Lyse der schwer abbaubaren Fraktionen zu gering,
- Thermische Desintegration als Zwischenstufe (FR 2 711 980 C2)
   * Vollstrombehandlung bei 80 bis 175 °C → sehr hoher Energieverbrauch und Abtötung der Methanbakterien,
- Entwässerung und Wärmebehandlung (EP 0 737 651 A1)
   * bei 60 °C oder höher → zu geringe Temperaturen für effektive Lyse.

In der WO 2006/042551 wird ein Verfahren zur Herstellung von Biogas aus organischem Abfall beschrieben, das folgende Schritte umfasst:
i) Fermentation des organischen Abfalls in einem ersten Biogasreaktor;
ii) Hydrolyse des aufgeschlossenen organischen Abfalls in einem anaeroben Hydrolysetank; und
iii) Fermentation des hydrolysierten organischen Abfalls in einem zweiten Biogasreaktor;
wobei die sich entwickelnden Gase aus dem anaeroben Hydrolysetank durch eine Gasdurchströmung im Kopf des Tanks entfernt werden.

Nachteile der Lösungen des Standes der Technik bestehen vor allem in der unzureichenden Gasausbeute im Vergleich zu der Gesamtverweilzeit der nachwachsenden Rohstoffe in der Biogasanlage.

Die Aufgabe der vorliegenden Erfindung besteht in der Angabe eines Verfahrens zur Konversion von Biomasse aus nachwachsenden Rohstoffen zu Biogas in anaeroben Fermentern, welches in kürzerer Gesamtverweilzeit der nachwachsenden Rohstoffe in der Biogasanlage und/oder eine höhere Quantität und/oder Qualität des Biogases realisiert.

Die Aufgabe wird durch die in den Ansprüchen angegebene Erfindung gelöst. Vorteilhafte Ausgestaltungen sind Gegenstand der Unteransprüche.

Bei dem erfindungsgemäßen Verfahren zur Konversion von Biomasse aus nachwachsenden Rohstoffen zu Biogas in anaeroben Fermentern werden nachwachsende Rohstoffe in einen mindestens ersten anaeroben Fermenter-Reaktor gemeinsam mit Flüssigkeit und weiteren für die Methanogenese notwendigen Ausgangsstoffen eingebracht und dort einem Gärprozess unterworfen, nachfolgend der Gärrest einer Fest-Flüssig-Phasentrennung unterzogen und die abgetrennte Feststoffphase einer Thermodruckhydrolyse bei Temperaturen von mindestens 170 °C und Drücken von mindestens 1,0 MPa unterworfen und die so behandelte Feststoffphase entweder in den ersten anaeroben Fermenter-Reaktor oder einem zweiten anaeroben Fermenter-Reaktor zugeführt und einem weiteren Gärprozess unterworfen, und mindestens Anteile der Flüssigphase nach der Fest-Flüssig-Phasentrennung dem ersten oder zweiten anaeroben Fermenter-Reaktor zugeführt.

Vorteilhafterweise werden als weitere für die Methanogenese notwendige Ausgangsstoffe Gülle und/oder Klärschlämme und/oder Prozesswässer zugegeben.

Ebenfalls vorteilhafterweise wird die Fest-Flüssig-Phasentrennung durch Pressen, Zentrifugation oder Siebung durchgeführt.

Weiterhin vorteilhafterweise wird die Thermodruckhydrolyse durch mechanische Bearbeitungsschritte der Feststoffphase vor oder während der Thermodruckhydrolyse ergänzt.

Vorteilhaft ist es auch, wenn die Thermodruckhydrolyse durch chemische und/oder enzymatische Bearbeitungsschritte der Feststoffphase vor oder während der Thermodruckhydrolyse ergänzt wird.

Und auch vorteilhaft ist es, wenn die Thermodruckhydrolyse bei Temperaturen im Bereich von 170 bis 250 °C durchgeführt wird.

Von Vorteil ist es ebenfalls, wenn die Thermodruckhydrolyse bei Drücken von 1 bis 4 MPa durchgeführt wird.

Und auch von Vorteil ist es, wenn die Thermodruckhydrolyse durch Nutzung der Abwärme anderer Prozesse durchgeführt wird.

Mit dem erfindungsgemäßen Verfahren wird es erstmals möglich, ein Biogaserzeugungsverfahren anzugeben, welches in kürzerer Zeit und/oder mit höherer Ausbeute Biogas herstellt. Dabei kann die Gesamtverweilzeit der Biomasse von bisher 50 bis 150 Tagen auf unter 50 Tage verkürzt werden. Gleichzeitig kann sowohl die Quantität als auch die Qualität des hergestellten Biogases durch einen Abbaugrad der eingesetzten Biomasse von über 80 % verbessert werden.

Mit dem erfindungsgemäßen Verfahren werden nachwachsende Rohstoffe zu Biogas verarbeitet. Unter nachwachsenden Rohstoffen sollen im Rahmen der vorliegenden Erfindung alle Rohstoffe verstanden werden, die zum Zweck der Energiegewinnung landwirtschaftlich angebaut werden. Weiterhin sollen ebenfalls unter diesen Begriff Reststoffe aus der landwirtschaftlichen Produktion verstanden werden.
Keine nachwachsenden Rohstoffe im Sinne der vorliegenden Erfindung sind Klärschlämme.

Der Unterschied zwischen den nachwachsenden Rohstoffen und Klärschlämmen für das erfindungsgemäße Verfahren ist auch insbesondere darin zu sehen, dass die nachwachsenden Rohstoffe in jeder Verfahrensstufe einen deutlich größeren Anteil an organischem Trockenrückstand, an Zellulose und Lignin sowie auch eine deutlich größere Partikelgröße vorweisen.

Mit dem erfindungsgemäßen Verfahren kann eine nahezu vollständige energetische Verwertung der eingesetzten Biomasse und deren Umsetzung in Biogas realisiert werden. Es kann nahezu die gesamte im Substrat enthaltene Primärenergie in Biogas umgewandelt werden, welches anschließend verstromt wird.

Auch können aufgrund der deutlich gesteigerten Abbaugeschwindigkeit die bestehenden Fermentervolumina wesentlich effektiver ausgenutzt und neu zu planende Anlagen kleiner dimensioniert werden.

Mit dem erfindungsgemäßen Verfahren wird eine annähernd vollständige Ausnutzung der Methanisierungskapazität der anaeroben Fermenter erreicht. Dies heißt, dass alle Fermenter-Reaktoren einer Biogasanlage für die Vergärung oder Mitvergärung von nachwachsenden Rohstoffen stets mit einer derart großen Menge an leicht umsetzbaren Substraten beschickt werden, dass es zu keiner ausgeprägten Geschwindigkeitslimitierung durch die Hydrolyse mehr kommt. Die zugeführten Stoffe werden so schnell umgesetzt, dass für die Methanstufe stets genügend Essigsäure zur Verfügung steht.

Bei einer durchschnittlichen Aufenthaltsdauer von 25 Tagen im ersten anaeroben Fermenter-Reaktor werden bekanntermaßen etwa 40 % der organischen Ausgangsstoffe abgebaut und in Biogas umgewandelt. Dabei arbeitet dieser erste anaerobe Fermenter-Reaktor mit voller Methanisierungsleistung, was durch einen in der Praxis festgestellten höheren Gehalt an organischen Säuren als Zwischenprodukt der anaeroben Abbaukette nachgewiesen worden ist. Besonders ein höherer Gehalt an Essigsäure weißt entweder auf eine Hemmung der Essigsäure verwertenden Methanogenen oder auf eine hohe Auslastung dieser Bakteriengruppe hin.

Um in dem nachgeschalteten zweiten anaeroben Fermenter-Reaktor Ausgangsstoffe mit einem ähnlich hohen Anteil an leicht verfügbare Substraten für einen Gärprozess bereitzustellen, werden erfindungsgemäß zwei Zwischenschritte realisiert. Zuerst wird der Gärrest des ersten anaeroben Fermenter-Reaktors einer Fest-Flüssig-Phasentrennung unterzogen. Der Gärrest besteht im Wesentlichen aus nichtabgebauten nachwachsenden Rohstoffen, Mikroorganismen und Wasser. Durch eine einfache Zentrifugalkraftentwässerung oder durch Abpressen werden das Wasser und die Mikroorganismen gemeinsam abgetrennt und können als Flüssigphase dem zweiten anaeroben Fermenter-Reaktor zugeführt oder als Abprodukt behandelt werden.
Die ebenfalls separierte Feststoffphase, die ca. 20 bis 30 % des Gesamtvolumens des Gärrestes des ersten anaeroben Fermenter-Reaktors darstellt, enthält im Wesentlichen das noch vorhandene Gasbildungspotential, welches durch eine nahezu vollständige Spaltung der sehr schwer hydrolysierbaren Makromoleküle im Thermodruckhydrolyseschritt im zweiten anaeroben Fermenter-Reaktor freigesetzt werden kann.

Der erfindungsgemäße Thermodruckhydrolyseprozess wird dann beispielsweise in einem zusätzlichen Reaktor oder auch in der Rohrleitung zwischen erstem und zweitem anaeroben Fermenter-Reaktor durchgeführt. Dazu kann vorteilhafterweise die Abwärme eines Blockheizkraftwerken eingesetzt werden. Die sehr großen Energiemengen werden der Feststoffphase beispielsweise mittels eines Abgaswärmetauschers zugeführt und die Feststoffphase im Chargenbetrieb bei mindestens 170 °C und 1,0 MPa zwischen 10 und 120 min der Thermodruckhydrolyse unterworfen. Eine zusätzliche Unterstützung der Thermodruckhydrolyse kann durch mechanische Bewegung der Feststoffphase erreicht werden. Durch die Thermodruckhydrolyse mit relativ hohen Temperaturen werden die in der Feststoffphase im Wesentlichen enthaltenen Zellulosen und Lignine aus dem Gärrest nach der Phasentrennung aufgeschlossen und in ihre Grundbestandteile (Monomere) zerlegt und damit für die weitere Umsetzung zu Biogas in einem Fermenter-Reaktor zugänglich gemacht. Dies ist bisher bei den Lösungen des Standes der Technik nicht möglich gewesen.
Die so behandelte Feststoffphase wird vollständig dem zweiten anaeroben Fermenter-Reaktor zugeführt, wo sie nahezu vollständig und mit vergleichsweise hoher Geschwindigkeit in Biogas umgewandelt wird.

Im Falle des Vorhandenseins nur eines anaeroben Fermenter-Reaktors, kann die der Thermodruckhydrolyse unterworfen Feststoffphase auch wieder in diesen Reaktor rückgeführt werden. Damit kann im Wesentlichen nur ein diskontinuierlicher Prozess durchgeführt werden. Ein Vorteil dieser Lösung ist jedoch die verminderte Menge an benötigter Frischbiomasse als Ausgangsstoff.
Das erfindungsgemäße Verfahren kann aber auch innerhalb einer Mehrzahl von Fermenter-Reaktoren realisiert werden, sofern diese beispielsweise bereits vorhanden sind.

Durch die Fest-Flüssig-Phasentrennung wird weiterhin der Vorteil erzielt, dass das Behandlungsvolumen deutlich reduziert wird, was eine Einsparung oder effektivere Ausnutzung der eingesetzten Wärmeenergie bedeutet. Weiterhin werden dadurch die im Gärrest nach dem ersten anaeroben Fermenter-Reaktor intakten Methanbakterien und anderen Mikroorganismen keiner Wärmebehandlung unterzogen und können weiterhin im ersten oder zweiten anaeroben Fermenter-Reaktor eingesetzt werden.
Und ein weiterer Vorteil besteht in der Erhöhung der hydraulischen Verweilzeit im zweiten Reaktor, da ein Teil der Flüssigphase aus dem Gesamtverfahren entzogen werden kann.

Ein weiterer Vorteil des erfindungsgemäßen Verfahrens besteht in seiner hohen Flexibilität, welche sich durch die Fest-Flüssig-Phasentrennung ergibt. Es können die Milieuverhältnisse des zweiten anaeroben Fermenter-Reaktors über die Veränderung der Flüssigphasenzugabe beeinflusst werden, was sich positiv auf die Betriebsstabilität und damit die Biogasausbeute auswirkt.

Nachfolgend wird die Erfindung an einem Ausführungsbeispiel näher erläutert.

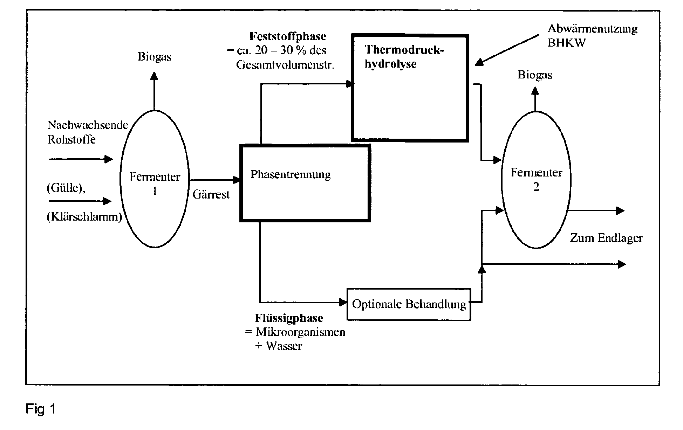

Dabei zeigt
- Fig. 1: das Verfahrensschema mit zwei Fermentern

50t Silage, bestehend aus 60%Mais und 40%Roggen werden täglich gemeinsam mit 100m³ Gülle einem kontinuierlich betriebenen, anaeroben Rührkesselreaktor als erstem Fermenter-Reaktor zugeführt. In dieser ersten Fermentationsstufe werden innerhalb von 20 Tagen hydraulischer Verweilzeit etwa 40% der im Input enthaltenen organischen Substrate zu Biogas umgesetzt.

Der Gärrest dieser ersten Stufe (ca. 136t/d) wird mit Hilfe einer Schneckenpresse entwässert. Dabei entstehen ca. 107t Flüssigphase mit einem Feststoffgehalt von 4% und ca. 31t Feststoffe mit einem Trockenrückstand von 30%. Zwei Drittel der Flüssigphase werden in den nachfolgenden Fermenter 2 überführt, das übrige Drittel zum Endlager (Stoffliche Verwertung). Die abgetrennten Feststoffe werden einer 30minütigen Behandlung bei 220°C und 2,5 MPa unterzogen. Dies erfolgt im Chargenbetrieb unter Nutzung der Abwärme eines Blockheizkraftwerkes (BHKW). Im Anschluss dieser Thermodruckhydrolyse gelangen die teilweise verflüssigten Feststoffe ebenfalls in den zweiten Fermenter-Reaktor. Die enthaltenen organischen Anteile werden im zweiten Fermenter-Reaktor während der hydraulischen Aufenthaltszeit von 20 Tagen nahezu vollständig in Biogas umgewandelt. Der erreichte Gesamtabbaugrad der organischen Substanz in beiden Fermentern beträgt insgesamt 80%.

## Patentansprüche

1. Verfahren zur Konversion von Biomasse aus nachwachsenden Rohstoffen zu Biogas in anaeroben Fermentern, bei dem nachwachsende Rohstoffe in einen mindestens ersten anaeroben Fermenter-Reaktor gemeinsam mit Flüssigkeit und weiteren für die Methanogenese notwendigen Ausgangsstoffen eingebracht und dort einem Gärprozess unterworfen werden, nachfolgend der Gärrest einer Fest-Flüssig-Phasentrennung unterzogen und die abgetrennte Feststoffphase einer Thermodruckhydrolyse bei Temperaturen von mindestens 170 °C und Drücken von mindestens 1,0 MPa unterworfen wird und die so behandelte Feststoffphase entweder in den ersten anaeroben Fermenter-Reaktor rückgeführt oder einem zweiten anaeroben Fermenter-Reaktor zugeführt wird und einem weiteren Gärprozess unterworfen wird, und bei dem mindestens Anteile der Flüssigphase nach der Fest-Flüssig-Phasentrennung dem ersten oder zweiten anaeroben Fermenter-Reaktor zugeführt werden.

2. Verfahren nach Anspruch 1, bei dem als weitere für die Methanogenese notwendige Ausgangsstoffe Gülle und/oder Klärschlämme und/oder Prozesswässer zugegeben werden.

3. Verfahren nach Anspruch 1, bei dem die Fest-Flüssig-Phasentrennung durch Pressen, Zentrifugation oder Siebung durchgeführt wird.

4. Verfahren nach Anspruch 1, bei dem die Thermodruckhydrolyse durch mechanische Bearbeitungsschritte der Feststoffphase vor oder während der Thermodruckhydrolyse ergänzt wird.

5. Verfahren nach Anspruch 1, bei dem die Thermodruckhydrolyse durch chemische und/oder enzymatische Bearbeitungsschritte der Feststoffphase vor der Thermodruckhydrolyse ergänzt wird.

6. Verfahren nach Anspruch 1, bei dem die Thermodruckhydrolyse bei Temperaturen im Bereich von 170 bis 250 °C durchgeführt wird.

7. Verfahren nach Anspruch 1, bei dem die Thermodruckhydrolyse bei Drücken von 1 bis 4 MPa durchgeführt wird.

8. Verfahren nach Anspruch 1, bei dem die Thermodruckhydrolyse durch Nutzung der Abwärme anderer Prozesse durchgeführt wird.

## Claims

1. Method for the conversion of biomass from renewable raw materials to biogas in anaerobic fermenters, in which renewable raw materials are introduced into an at least first anaerobic fermenter reactor together with liquid and further starting materials necessary for methanogenesis and are there subjected to a fermentation process, subsequently the fermentation residue is subjected to a solid-liquid phase separation and the solid phase that is separated off is subjected to a thermal and pressure hydrolysis at temperatures of at least 170°C and pressures of at least 1.0 MPa, and the solid phase that is thus treated is either recirculated to the first anaerobic fermenter reactor or fed to a second anaerobic fermenter reactor and subjected to a further fermentation process, and in which at least some of the liquid phase after the solid-liquid phase separation is fed to the first or second anaerobic fermenter reactor.

2. Method according to Claim 1, in which, as further starting materials necessary for methanogenesis, liquid manure and/or sewage sludges and/or process waters are added.

3. Method according to Claim 1, in which the solid-liquid phase separation is carried out by pressing, centrifugation or screening.

4. Method according to Claim 1, in which the thermal and pressure hydrolysis is supplemented by mechanical processing steps of the solid phase before or during the thermal and pressure hydrolysis.

5. Method according to Claim 1, in which the thermal and pressure hydrolysis is supplemented by chemical and/or enzymatic processing steps of the solid phase before the thermal and pressure hydrolysis.

6. Method according to Claim 1, in which the thermal and pressure hydrolysis is carried out at temperatures in the range from 170 to 250°C.

7. Method according to Claim 1, in which the thermal and pressure hydrolysis is carried out at pressures from 1 to 4 MPa.

8. Method according to Claim 1, in which the thermal and pressure hydrolysis is carried out by utilizing the waste heat of other processes.

## Revendications

1. Procédé pour la conversion d'une biomasse à base de matières premières renouvelables en un biogaz dans des fermenteurs anaérobies, dans lequel des matières premières renouvelables sont introduites dans au moins un premier réacteur de fermentation anaérobie avec un liquide et d'autres matières premières nécessaires pour la méthanogenèse, et y sont soumises à un procédé de fermentation, puis le résidu de fermentation est soumis à une séparation de phases solide-liquide et la phase solide séparée est soumise à une hydrolyse sous thermopression à des températures d'au moins 170 °C et des pressions d'au moins 1,0 MPa, et la phase solide ainsi traitée est recyclée dans le premier réacteur de fermentation anaérobie ou introduite dans un second réacteur de fermentation anaérobie et soumise à un procédé de fermentation supplémentaire, et dans lequel au moins des fractions de la phase liquide après la séparation de phases solide-liquide sont introduites dans le premier ou le second réacteur de fermentation anaérobie.

2. Procédé selon la revendication 1, dans lequel du purin et/ou des boues d'épuration et/ou des eaux de fabrication sont ajoutés en tant qu'autres matières premières nécessaires pour la méthanogenèse.

3. Procédé selon la revendication 1, dans lequel la séparation de phases solide-liquide est réalisée par compression, centrifugation ou tamisage.

4. Procédé selon la revendication 1, dans lequel l'hydrolyse sous thermopression est complétée par des étapes de traitement mécanique de la phase solide avant ou pendant l'hydrolyse sous thermopression.

5. Procédé selon la revendication 1, dans lequel l'hydrolyse sous thermopression est complétée par des étapes de traitement chimique et/ou enzymatique de la phase solide avant l'hydrolyse sous thermopression.

6. Procédé selon la revendication 1, dans lequel l'hydrolyse sous thermopression est réalisée à des températures dans la plage allant de 170 à 250 °C.

7. Procédé selon la revendication 1, dans lequel l'hydrolyse sous thermopression est réalisée à des pressions de 1 à 4 MPa.

8. Procédé selon la revendication 1, dans lequel l'hydrolyse sous thermopression est réalisée en utilisant la chaleur d'échappement d'autres procédés.
